Europäisches Patentamt

European Patent Office

Office européen des brevets

(19)

(11) Veröffentlichungsnummer : **0 149 126**
**B1**

(12) **EUROPÄISCHE PATENTSCHRIFT**

(45) Veröffentlichungstag der Patentschrift :
**19.08.87**

(21) Anmeldenummer : **84115066.7**

(22) Anmeldetag : **10.12.84**

(51) Int. Cl.⁴ : **A 61 B 6/04**, F 16 H 21/22

(54) **Röntgendiagnostikeinrichtung mit einer Drehmulde.**

(30) Priorität : **19.01.84 DE 3401788**

(43) Veröffentlichungstag der Anmeldung :
**24.07.85 Patentblatt 85/30**

(45) Bekanntmachung des Hinweises auf die Patenterteilung : **19.08.87 Patentblatt 87/34**

(84) Benannte Vertragsstaaten :
**DE FR**

(56) Entgegenhaltungen :
**DE-A- 1 949 763**
**US-A- 3 757 129**
**US-A- 3 848 132**
**ENGINEERING, Band 218, Nr. 9, September 1978,**
**Seiten 880-884, London, GB; G. ROONEY: "Mechanis-**
**ms: Geared linkages"**

(73) Patentinhaber : **Siemens Aktiengesellschaft Berlin**
**und München**
**Wittelsbacherplatz 2**
**D-8000 München 2 (DE)**

(72) Erfinder : **Goldhorn, Klaus, Dipl.-Ing.**
**Ganghoferweg 2**
**D-8520 Erlangen (DE)**

**Beschreibung**

Die Erfindung betrifft eine Röntgendiagnostikeinrichtung, die Mittel zur Strahlenerzeugung, eine Aufnahmevorrichtung mit einer Bildschichtebene und eine Drehvorrichtung für einen Patienten mit Antriebs- und Führungsmitteln aufweist, durch die die Drehvorrichtung während ihrer Drehung eine Hubbewegung senkrecht zur Bildschichtebene ausführt, so daß der Abstand zwischen der Bildschichtebene und dem jeweils bildschichtnächsten Punkt der Oberfläche der Drehvorrichtung etwa konstant bleibt. Hierbei weisen die Führungsmittel einen an der Drehvorrichtung befestigten Zapfen auf, der in einem sich in Hubrichtung erstreckenden Langloch eines gerätefesten Lagerbockes geführt ist. Es ist eine in einem gerätefesten Lager drehbar gelagerte Antriebswelle vorhanden, die mit einem auf ihr befestigten Ritzel in eine mit der Drehvorrichtung fest verbundene Verzahnung eingreift. Eine derartige Drehvorrichtung kann beispielsweise eine ovale Drehmulde als Patientenlagerstatt aufweisen, die stirnseitig am Gerätekörper gelagert ist.

Aus der DE-PS 19 49 763 ist eine derartige Röntgendiagnostikeinrichtung bekannt, bei der die Drehung der Drehmulde durch eine spezielle Triebstockverzahnung erfolgt, in die ein Antriebsritzel eingreift. Aufgrund der speziellen Form einer Triebstockverzahnung läuft das Antriebsritzel in einer Führungsbahn, die neben einer Drehung ein Anheben der Drehmulde bewirkt. Eine derartige Einrichtung weist einen unruhigen Bewegungsablauf auf. Weiterhin ist die Triebstockverzahnung in ihrer Herstellung sehr kosten- und arbeitsintensiv.

Die Erfindung geht von der Aufgabe aus, eine Röntgendiagnostikeinrichtung der eingangs genannten Art zu schaffen, bei der der Aufbau der Antriebsmittel bei ruhigem Bewegungsablauf einfach und kostengünstig ist.

Diese Aufgabe ist erfindungsgemäß dadurch gelöst, daß die Antriebswelle exzentrisch mit dem Ritzel derart verbunden ist, daß Ritzelachse und Drehachse der Welle parallel im Abstand voneinander liegen, und daß exzentrisch auf dem Zapfen ein Zahnrad befestigt ist, das in das Antriebsritzel eingreift. Durch diesen Aufbau wird erreicht, daß durch Drehung der Antriebswelle zum einen das Zahnrad angehoben und zum anderen verdreht wird. Durch die Exzentrizität des Zahnrades wird diese Dreh- und Hubbewegung des im Drehpunkt der Drehmulde befindlichen Zapfens noch verstärkt. Es hat sich als vorteilhaft erwiesen, wenn zur Sicherung der Drehvorrichtung gegen Abheben des Zahnrades vom Ritzel eine Verbindungsstange drehbar mit deren Achsen verbunden ist.

Die Erfindung ist nachfolgend anhand eines in der Zeichnung dargestellten Ausführungsbeispieles näher erläutert. Es zeigen:

Figur 1 eine perspektivische Ansicht einer Röntgendiagnostikeinrichtung mit einer Drehmulde nach der Erfindung,

Figur 2 eine Seitenansicht einer Stirnseite der Drehmulde in ihrer horizontalen Ausgangsstellung mit der zugehörigen Lagerungs- und Antriebsvorrichtung,

Figur 3 eine Schnittansicht längs der Linie III-III in Figur 2 mit leicht verdrehter Drehmulde, und

Figur 4 eine Ansicht gemäß Figur 3 bei um 90° verdrehter Drehmulde.

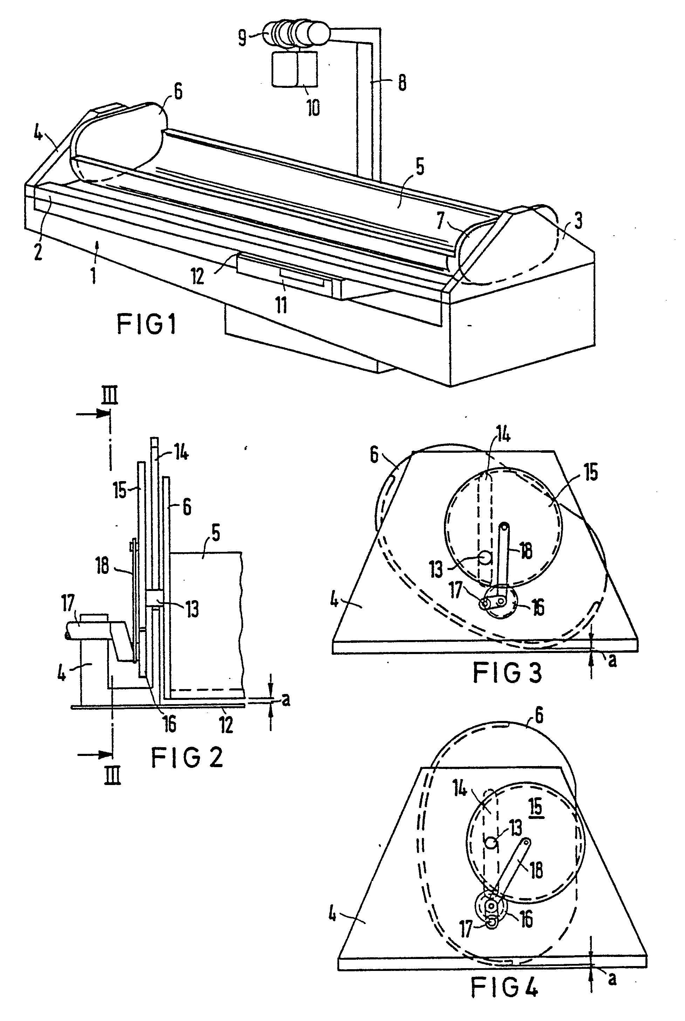

In der Figur 1 ist eine Röntgendiagnostikeinrichtung 1 mit einem Tragrahmen 2 und zwei Lagerböcken 3 und 4 dargestellt, in denen eine Drehmulde 5 mit einer Kopfplatte 6 und einer Fußplatte 7 verdrehbar gelagert ist. An einem Stativ 8 ist oberhalb der Drehmulde 5 eine Röntgenröhre 9 mit einer Primärstrahlenblende 10 angebracht. Unterhalb dieser Drehmulde 5 befindet sich innerhalb des Strahlenganges der Röntgenröhre 9 eine Aufnahmevorrichtung, die in diesem Falle aus einer Kassettenhaltevorrichtung 11 mit einer Bildschichtebene 12 gebildet ist. Es läßt sich aber auch eine Röntgenbildverstärker-Fernsehkette verwenden.

In der Figur 2 sind die Antriebs- und Führungsmittel dargestellt, die der Kopfplatte 6 der Drehmulde 5 zugeordnet sind. Durch einen Zapfen 13 ist die Drehmulde 5 in einem Langloch 14 des Lagerbockes 4 geführt. An dem Zapfen 13 ist exzentrisch ein Zahnrad 15 angebracht, das auf einem Ritzel 16 aufliegt und in dessen Verzahnung eingreift. Das Ritzel 16 ist mit einer Antriebswelle 17 fest verbunden, die im Lagerbock 4 gelagert und direkt oder über ein Getriebe mit einem nicht dargestellten Motor verbunden ist. Zwischen der Lagerung im Lagerbock 4 und dem Ritzel 16 ist die Antriebswelle 17 zweifach gekröpft, so daß die Achse des Ritzels 16 und die Achse der Antriebswelle 17 parallel im Abstand voneinander liegen. Damit in keiner Stellung der Drehmulde 5 das Zahnrad 15 von dem Ritzel 16 abheben kann, sind die Mittelpunkte der Zahnräder 15 und 16 durch eine drehbar befestigte Verbindungsstange 18 gehalten. In dieser horizontalen Ausgangsstellung weisen die beiden exzentrischen Drehpunkte der beiden Zahnräder 15 und 16, der Zapfen 13 und die Antriebswelle 17, ihren geringsten Abstand zueinander auf. Die Drehmulde 5 befindet sich von der Bildschichtebene 12 in einem Abstand a, der auch während der Drehung der Drehmulde 5 beibehalten werden soll.

Anhand der Figuren 3 und 4, in denen eine Draufsicht auf die Führungs- und Antriebsmittel der Drehvorrichtung nach dem Lager der Antriebswelle 17 dargestellt ist, wird die Funktionsweise noch weiter verdeutlicht. Das mit der gekröpften Antriebswelle 17 fest verbundene Ritzel 16 vollführt durch Drehung der Antriebswelle 17 eine Dreh- und eine Hubbewegung. Dadurch werden das Zahnrad 15 und damit die Drehmulde 5 ebenfalls verdreht und gleichzeitig angehoben. Durch die exzentrische Verbindung des Zahnrades 15 mit der Drehmulde 5 durch den exzentrisch an dem Zahnrad 15 angebrachten Zapfen 13 wird durch die Drehung des Zahnrades 15 die Drehmulde 5 zusätzlich angehoben. Dadurch wird der durch die Antriebswelle 17 durchgeführte Hub noch vergrößert, wie dies insbesondere in der Figur 4 bei der um 90° geschwenkten Drehmulde 5 in der Figur 4 gezeigt wird. Der Abstand a der Drehmulde 5 zur Bildschichtebene ist trotz Drehung durch die gleichzeitige Anhebung im wesentlichen gleich geblieben. Die Verbindungsstange 18, die mit den Achsen der Zahnräder 15 und 16 drehbar verbunden ist, hält beide Zahnräder 15 und 16 derart, daß deren Verzahnungen in jeder Stellung ineinandergreifen.

Da die Antriebs- und Führungsmittel symmetrisch aufgebaut sind, erfolgen die gleichen Dreh- und Hubbewegungen der Drehmulde 5 auch in anderer Drehrichtung. Es kann also durch die dargestellte Ausführungsform eine Drehung der Drehmulde 5 um ± 90° erfolgen. Durch Veränderung der Exzentrizitäten, der Zahnraddurchmesser und insbesondere der Zahnradübersetzung lassen sich aber auch Drehungen um 360° erreichen. Hierbei muß aber die Drehmulde derart ausgebildet sein, daß der untersuchte Patient gehalten wird.

In gleicher Weise wie die Führungs- und Antriebsmittel, die der Kopfplatte 6 zugeordnet sind, sind auch die der Fußplatte 7 zugeordnete Führungs- und Antriebsmittel ausgebildet. Der in dem Lagerbock 3 angeordnete Antrieb kann mechanisch über ein Getriebe mit dem Motor der Antriebsmittel für den Lagerbock 4 verbunden sein oder durch einen zweiten Elektromotor erfolgen, der mit dem ersten elektrisch gekoppelt ist. Für kleine und leichte Patienten, beispielsweise Babys und Kleinkinder, ist aber auch die Verwendung einer kleinen Mulde mit nur einseitiger Lagerung und einseitigem Antrieb möglich.

Die erfindungsgemäße Drehvorrichtung läßt sich aber auch bei einer Röntgendiagnostikeinrichtung mit beispielsweise horizontaler Strahlung einsetzen, wenn der zu untersuchende Patient auf einem Hocker sitzt, der sich auf einem entsprechend ausgebildeten Drehteller befindet. Dadurch kann der Patient mittels Fernsteuerung auf einfache Weise gedreht werden, so daß sich ein erneutes Ausrichten des Patienten erübrigt.

**Patentansprüche**

1. Röntgendiagnostikeinrichtung, die Mittel zur Strahlenerzeugung (9, 10), eine Aufnahmevorrichtung (11) mit einer Bildschichtebene (12) und eine Drehvorrichtung für einen Patienten mit Antriebs- und Führungsmitteln (13 bis 18) aufweist, durch die die Drehvorrichtung während ihrer Drehung eine Hubbewegung senkrecht zur Bildschichtebene (12) ausführt, so daß der Abstand (a) zwischen der Bildschicht und dem jeweils bildschichtnächsten Punkt der Oberfläche der Drehvorrichtung etwa konstant bleibt, bei der die Führungsmittel (13 bis 16) einen an der Drehvorrichtung befestigten Zapfen (13) aufweisen, der in einem sich in Hubrichtung erstreckenden Langloch (14) eines gerätefesten Lagerbockes (3, 4) geführt ist, und bei der eine in einem gerätefesten Lager drehbar gelagerte Antriebswelle (17) vorhanden ist, die mit einem auf ihr befestigten Ritzel (16) in eine mit der Drehvorrichtung fest verbundene Verzahnung eingreift, dadurch gekennzeichnet, daß die Antriebswelle (17) exzentrisch mit dem Ritzel (16) derart verbunden ist, daß Achse des Ritzels (16) und Drehachse der Antriebswelle (17) parallel im Abstand voneinander liegen, und daß exzentrisch auf dem Zapfen (13) ein Zahnrad (15) befestigt ist, das in das Antriebsritzel (16) eingreift.

2. Röntgendiagnostikeinrichtung nach Anspruch 1, dadurch gekennzeichnet, daß zur Sicherung der Drehvorrichtung gegen Abheben des Zahnrades (15) vom Ritzel (16) eine Verbindungsstange (18) drehbar mit deren Achsen verbunden ist.

**Claims**

1. An X-ray diagnostic apparatus which includes radiation generating means (9, 10), a supporting device (11) comprising a tomograph plane (12), and a patient rotating device comprising drive and guide means (13-18) by which, during its rotation, the rotating device executes an elevating movement at right-angles to the tomograph plane (12) so that the distance (a) between the tomograph and that point of the surface of the rotating device which is closest to the tomograph remains approximately constant, where the guide means (13-16) are provided with a pin (13) which is attached to the rotating device and which moves in a slot (14) extending in the elevating direction of a bearing block (3, 4) fixed to the apparatus and where a drive shaft (17) is provided which is mounted so as to be rotatable in a bearing fixed to the apparatus and which by means of an attached pinion (16) engages a toothed device which is permanently connected to the rotating device, characterised in that the drive shaft (17) is eccentrically connected to the pinion (16) in such a manner that the axis of the pinion (16) and the rotation axis of the drive shaft (17) are arranged in parallel to one another and that a gear wheel (15), which engages into the drive pinion (16), is attached eccentrically to the pin (13).

2. An X-ray diagnostic apparatus as claimed in claim 1, characterised in that to prevent the rotating device from lifting the gear wheel (15) from the pinion (16), a connecting rod (18) is rotatably connected to the axes thereof.

**Revendications**

1. Appareil de radiodiagnostic, qui comporte des moyens (9, 10) pour produire un rayonnement, un dispositif d'enregistrement (11) comportant un plan (12) de la couche image et un dispositif rotatif pour un patient comprenant des moyens d'entraînement et de guidage (13 à 18), à l'aide desquels le dispositif rotatif exécute, pendant cette rotation, un mouvement de levage perpendiculairement au plan (12) de la couche image de sorte que la distance (a) entre la couche image et le point respectif de la surface du dispositif rotatif, qui est plus proche de la couche image, reste approximativement constante, et dans lequel les moyens de guidage (13 à 16) comprennent un téton (13) fixé au dispositif rotatif et qui est guidé dans un trou allongé (14), s'étendant dans la direction de levage, d'un bloc de palier (3, 4) monté fixe dans l'appareil, et dans lequel il est prévu un arbre d'entraînement (17) monté de façon à pouvoir tourner dans un palier monté fixe dans l'appareil, et qui s'engage, par un pignon (16), fixé sur lui, dans une denture reliée rigidement au dispositif rotatif, caractérisé par le fait que l'arbre d'entraînement (17) est relié de façon excentrée au pignon (16) de telle sorte que l'axe du pignon (16) et l'axe de rotation de l'arbre d'entraînement (17) sont parallèles, à une certaine distance réciproque et que sur le téton (13) se trouve fixé, de façon excentrée, une roue dentée (15) qui engrène avec le pignon d'entraînement (16).

2. Appareil de radiodiagnostic suivant la revendication 1, caractérisé par le fait que pour protéger le dispositif rotatif vis-à-vis d'un écartement de la roue dentée (15) par rapport au pignon (16), une barre de liaison (18) est reliée, de façon à pouvoir tourner, aux axes de la roue dentée et du pignon.

FIG 1

FIG 2

FIG 3

FIG 4